Europäisches Patentamt

European Patent Office

Office européen des brevets

(11) Publication number: **0 687 471 A1**

# EUROPEAN PATENT APPLICATION

(21) Application number: **95108593.5**

(22) Date of filing: **06.06.95**

(51) Int. Cl.6: **A61K 39/002**, C12N 1/10,
G01N 33/569

(30) Priority: **17.06.94 US 261555**

(43) Date of publication of application:
**20.12.95 Bulletin 95/51**

(84) Designated Contracting States:
**AT BE CH DE DK ES FR GB GR IE IT LI NL PT
SE**

(71) Applicant: **Bayer Corporation
One Mellon Center
500 Grant Street
Pittsburgh, PA 15219-2502 (US)**
Applicant: **PARAVAX, INC.
1825 Sharp Point Drive
Fort Collins, CO 80525 (US)**

(72) Inventor: **Popiel, Irene
3733 LaMesa Drive
Ft.Collins, CO 80524 (US)**
Inventor: **Gold, Marielle C.
729 Ponderosa Dr.
Fort Collins, CO 80521 (US)**
Inventor: **Choromanski, Leszek J.
14413 W. 80 Terrace
Lenexa, KS 66215 (US)**
Inventor: **Brown, Karen K.
5501 NW Fox Hill Rd.
Parkville, MO 64152 (US)**

(74) Representative: **Schumacher, Günter, Dr. et al
Bayer AG
Konzernzentrale RP
Patente Konzern
D-51368 Leverkusen (DE)**

(54) Production of an efficacious toxoplasma gondii bradyzoite vaccine in tissue culture

(57) Disclosed herein is a tissue culture produced vaccine for protection of cats against T. gondii, containing an effective amount of bradyzoites and characterized in that the vaccine is substantially free of tachyzoites. The vaccine can be produced on a commercial scale by growing a mutant of T. gondii in a tissue culture to produce bradyzoites by adding T. gondii positive antisera and proteolytic enzymes to inhibit growth or kill residual tachyzoites.

EP 0 687 471 A1

## BACKGROUND OF THE INVENTION

Field of the Invention:

The present invention relates to toxoplasmosis. More specifically the invention relates to tissue cultures of Toxoplasma gondii (T. gondii), vaccines and diagnostic kits prepared with them, their methods of preparation and use.

Brief Description of the Prior Art:

Toxoplasmosis is an important parasitic disease of humans and domestic animals. It is reported as a serious problem for seronegative pregnant women who, if infected during pregnancy, may bear a malformed or dead fetus. It is also reported as a serious problem for immunocompromised individuals such as AIDs patients and cancer patients on certain immunosuppressive drug therapies. Many of these individuals, reportedly die of toxoplasmosis.

Cats are the only animals that are known to spread the disease via shedding of oocysts. Upon the first exposure via consumption of T. gondii-infected mammals or birds, cats show no signs of the disease but shed as many as 100 million fresh oocysts in their feces. The oocysts may remain in moist and shaded soil for up to 18 months and stay infective to humans and other animals during this time. The oocysts can infect other mammals that contact them, particularly via the oral route. Cats become infected by ingesting infected small mammals and birds that carry cysts or **oocysts** in their tissues. Cats are, therefore, the definitive hosts in the complex life of T. gondii. A sexual replicative cycle of T. gondii occurs within the guts of cats and results in the oocysts that the cats shed in their feces

Oocysts are infective to other mammals which usually become infected via the oral route. In these other mammals, a rapidly dividing stage of T. gondii that is called the tachyzoite can cause an acute infection of tissues. A more latent slowly dividing stage that is called the bradyzoite develops from tachyzoites. Bradyzoites develop within the tissue cysts (protective shells). Thousands of bradyzoites may develop within one tissue cyst. These tissue cysts are found in skeletal and heart muscle, brain and other tissues of the central nervous system. Of particular concern here is the infection of humans either by direct contact with oocysts in soil and litter boxes, or by eating undercooked meat of infected animals containing infective tissue cysts. It is of note that while other mammals do not support the sexual replicative cycle of T. gondii, they can, nonetheless, be infected by T. gondii. The infection results in a systemic parasitemia and formation of tissue cysts.

As would be realized from the foregoing, a vaccine that can protect cats from infection and subsequent shedding of oocysts would be a valuable contribution to human health. Drs. J. Frenkel and E. Pfefferkorn (U.S. Patent 4,473,549) have identified an oocyst negative mutant of T. gondii, designated T-263. Oral administration of tissue cysts of T. gondii to cats induces immunity to oocyst shedding, following challenge.

Since tachyzoites are easily grown in tissue cultures, a vaccine prepared from tachyzoites would be ideal. However, live or inactivated vaccines containing tachyzoites of the T-263 mutant have not been found to protect cats. More specifically, they do not protect cats from shedding oocysts after exposure to wild-type strains. It is of note that even if the tachyzoites are surgically administered into the duodenum, they do not protect cats from the shedding of oocysts after challenge with a wild type oocyst shedding strain that is designated as T-265.

In contrast to tachyzoites, bradyzoites of the modified-live T-263 mutant produced in mice have been shown to protect cats from oocyst shedding (Frenkel, U.S. Patent 4,473,549), as follows. While the modified live T-263 strain was unable to produce oocysts (negative phenotype) when administered to cats, it protected against oocysts shedding, if the cats had been orally vaccinated with T-263 and then exposed to a virulent oocyst-producing strain of T.gondii.

Although the bradyzoites of T-263 are apparently effective in vaccines, there are no reports of commercial scale tissue culture production of bradyzoites. The current knowledge of tissue culture growth of bradyzoites involves laboratory scale growth of tachyzoites, under special conditions and converting them to bradyzoites in tissue cysts. Lindsay, et al (J. Parasitology, 1991 77(l), pages 126-132) discloses tissue cyst formation with a goat isolate of T. gondii in the following cells: bovine monocytes, human fetal lung cells and Madin-Darby bovine kidney cells. However, cats fed with preparations containing these tissue culture grown bradyzoites shed oocysts in their feces. The disclosure of Lindsey et al teaches the use the tissue culture grown T. gondii to test drugs for activity against this organism. It does not teach the use of tissue culture derived tissue cysts to develop a vaccine. It does not teach the inhibition of the growth of tachyzoites by the use of antibodies or other means.

Jones et al (Infection and Immunity, Jan. 1986, pages 147-156) discloses in vitro cultivation of T. gondii cysts in mouse astrocyte cells. The disclosure teaches the use of gamma interferon to inhibit overgrowth of tissue culture cells with tachyzoites to allow the development of tissue cysts. It does not teach the use of antibodies or enzymes. Also, it does not teach the use of tissue culture-grown tissue cysts for production of a vaccine. Notably the disclosure states that one would require 40-80 days to form a significant number of tissue cysts. The cost of such long term growth would be prohibitive in the commercial production of vaccines.

Shimada et al (Arch Ophthalmol., Vol 92, Dec. 1974) discloses that the RH strain of T. gondii can grow in rabbit corneal endothelial cells in Leighton tubes. The disclosure does teach the use of an antibody and a guinea pig complement to inhibit growth of tachyzoites. It teaches that the use of an immune serum alone or guinea pig complement alone minimally inhibited growth of tachyzoites, and that their combination showed a synergistic effect. The disclosure does not teach the use of enzymes to kill tachyzoites or the use of tissue cysts for the preparation or testing of vaccines.

As would be realized, production of an efficacious and cost-effective tissue culture based T. gondii vaccine containing bradyzoites would be desirable. Heretofore, T. gondii bradyzoites have not been produced on a commercial scale in tissue culture, formulated tested in vaccines and shown to be protective when used to vaccinate cats.

SUMMARY OF THE INVENTION

In accordance with the foregoing, the present invention encompasses a tissue culture produced T. gondii containing an immunogenically effective amount of bradyzoites for protection of cats. By the term "immunogenically effective amount" is meant the amount of bradyzoites in a vaccine that will protect cats from shedding of oocysts. In the present embodiment of the invention, as few as 10 modified live bradyzoites administered orally to cats can provide protection.

By the present invention one can obtain a commercial scale tissue culture containing T. gondii bradyzoites. The term "commercial scale" means that the T. gondii bradyzoites are produced in such a high yield that vaccines can be formulated therewith without an appreciable and/or costly concentration of the antigenic material of the tissue culture. As would be realized, concentration of the antigenic material can damage live bradyzoites. In art-related processes such as laboratory scale tissue culture growth of T. gondii, the antigenic material would have to be concentrated appreciably to produce vaccines therewith.

Also encompased by the invention is a process for preparing a tissue culture of T. gondii comprising: growing tachyzoites of the T. gondii in the tissue culture, keeping the tissue culture from being overgrown by tachyzoites and **developing** bradyzoites in tissue cysts, and killing residual tachyzoites.

Further encompassed by the invention are the vaccines or diagnostic kits containing organisms obtained by the claimed invention.

The method of administering the vaccine to cats to protect them against oocyst shedding is also encompassed by the invention. The invention is described more fully hereunder.

DETAILED DESCRIPTION OF THE INVENTION

As set forth above, the present invention is directed to a tissue culture produced T. gondii that contains an immunogenically effective amount of bradyzoites for protection of cats against T. gondii. Any tissue culture primary cell or cell line which supports the growth of T. gondii may be used. Illustratively, the tissue culture cell or cell line can be selected from the group consisting of: embryonic bovine lung cells, mouse astrocyte cells, a Human Foreskin Fibroblast cell line and a Human Diploid Lung cell (MRC-5). Also, the Crandell cell line (feline) Cutter Laboratories Dog Kidney Cell Line (CLDK), Vero Cell Line and Madin-Darby Bovine Kidney (MDBK) cell lines have been demonstrated to grow the organism. Slow-growing cells such as the embryonic bovine lung cell, are particularly useful in growing T. gondii bradyzoites.

The T. gondii useful herein is that which contains protective antigen and lacks reproductive capability. By the term "lacks reproductive capability" is meant the T. gondii strain cannot produce oocysts which could be shed to the environment. An illustrative but nonlimiting example of the T. gondii useful herein can be a T-263 mutant that has been deposited with the American Type Culture Collection and has been accorded ATCC Accession No. 40615.

Modified-live or attenuated mutants may be produced by exposing the tachyzoites of T. gondii strain such as the "C" strain to an alkylating agent, such as N-methyl-N-nitro-nitroso-guanidine [Pfefferkron, E.R. and Pfefferkron, L.C., "Toxoplasma gondii: Isolation and Preliminary Characterization of Temperature Sensitive Mutants." Experimental Parasitology 39, 365-376, (1976)], and isolating mutants which are resistant to one or more antimetabolites. The resistant mutants are then selected further for the oocyst negative phenotype and, finally, selected for their ability to protect cats.

In a specific embodiment, the process for preparing the tissue culture of T. gondii comprises: growing T. gondii tachyzoites in a tissue culture; keeping the tissue culture from being overgrown by the tachyzoites and developing therefrom bradyzoites in tissue cysts by addition of T. gondii-specific antibody (positive antisera); propagating bradyzoites to a high yield which is sufficient to produce an immunogenically effective amount of bradyzoites, and reducing the number of residual tachyzoites by addition of enzymes that are lethal to tachyzoites. **Hence, the tissue culture is characterized in that it is substantially free of live tachyzoites.** The process results in a high yield of bradyzoites for the preparation of vaccines on a commercial scale. Without being bound to any particular theory of the invention, it is envisaged that the T. gondii tachyzoite-infected tissue culture is stimulated to produce bradyzoites in tissue cysts by the addition of the positive antisera.

The following is a more specific but non-limiting description of the process for preparing the T. gondii in tissue culture. The tachyzoites can be grown in tissue culture systems which are typically closed systems. When the tachyzoites have grown to a sufficiently high level such that a typical cytopathic effect is observed, T. gondii-specific antibody (positive antisera) is added to the culture system. The positive antisera can be employed in a concentration that is effective in keeping the tissue culture from being overgrown by tachyzoites. Concentration of 0.1% to 10% and preferably from 3% to 5% can be employed. Illustrative examples of the positive antisera can be selected from but not limited to the group consisting of goat, rabbit, horse and bovine. The tissue cysts containing bradyzoites are allowed to develop until maximum numbers of tissue cysts are obtained. At this stage, the proteolytic enzyme is added to the tissue culture in an amount sufficient to substantially eliminate the residual tachyzoites. The addition of the proteolytic enzyme also breaks open the tissue cysts and causes the release of bradyzoites. The proteolytic enzyme can be employed in a concentration from 0.0001% to 5% and preferably from 0.0001% to 0.1% w/v. The proteolytic enzyme can be selected from the group consisting of pepsin, trypsin and chymotrypsin: pepsin is preferred.

For preparations on a commercial scale, **a tissue culture system** such as a roller bottle, bioreator or the like are employed. Illustratively, one roller bottle containing from 200 mL to 1L of tissue culture media can produce as many as 500 doses of vaccine. The time required for growth is approximately seven days.

It is a feature of the invention that in using large scale systems that are closed systems to grow the T. gondii bradyzoites, one does not use syringes and needles in the inoculation of cells. The closed systems therefore protect against infection of humans. It is also a feature of the invention that in using tissue culture to grow the T. gondii, the concentration of reactive components of the vaccines can be controlled. In essence, the tissue culture can be grown to be free of living contaminants (bacteria, viruses, fungi, etc.) and can be essentially free of reactive components.

Products containing the tissue culture can be vaccines, diagnostic kits or the like. A vaccine containing the tissue culture can be formulated by art-known formulating techniques. The resulting vaccine comprises T. gondii in a stage that is effective to protect cats against T. gondii. In the present embodiment of the invention, the vaccine comprises T. gondii bradyzoites. The vaccine can be in live or modified live form, and can be in a liquid, frozen or lyophilized state. The diagnostic kit can be prepared by art-known techniques.

The invention is further illustrated but is not intended to be limited by the following examples in which all parts and percentages are by weight unless otherwise specified.

EXAMPLES

A specific example of tissue culture growth of T-263 tissue cysts containing bradyzoites is described as follows. A vial of working seed tachyzoites originating from Master Seed produced in mouse peritoneal exudates and containing $5 \times 10^5$/mL tachyzoites was thawed and added to a confluent Human Foreskin Fibroblast (HSF) monolayer culture. These were allowed to progress until the cytopathic effect (CPE) was complete. This first tissue culture passage was conducted to adapt tachyzoites to tissue culture. Embryonic Bovine Lung (EBL) cells were grown in 75 cm$^2$ flasks using Dulbeccos Minimal Essential Medium DMEM + 10% Fetal bovine Serum (FBS) + glutamine and gentamicin. When the cells were confluent, the medium was removed and 12 ml/flask of Toxoplasma Maintenance Medium (TMM = DMEM with 4500 mg/mL glucose, 3% FBS, 1% glutamine at 2mM and 50 mg/mL gentamicin) was added. This was followed by addition of $5 \times 10^5$ tachyzoites from the HSF cell culture. The flasks were incubated at 37°C in an atmosphere of 5% $CO_2$ for 4 days. On day 4, fresh TMM plus 5% goat or rabbit antitoxoplasma serum determined to be positive by ELISA was added to each flask. Flasks were incubated 3 more days under the previously described conditions. On day 7, the cysts were harvested by scraping the cells off with a rubber policeman. (other similar means for cell removal, e.g., glass beads or enzymes (i.e., trypsin) which will not be detrimental to bradyzoites can be used) Using this method, there were obtained approximately 200 cysts with approximately 100,000 bradyzoites per flask, as determined by visual observation.

These live tissue cysts containing bradyzoites were used to prepare a vaccine. Vaccines were prepared in which the tissue cysts were left intact (NON PEPSIN TREATED) or were digested for 10 min. at 37°C with 0.05% pepsin (PEPSIN DIGESTED). The latter treatment not only killed the remaining tachyzoites but also released the bradyzoites. The vaccine formulations were diluted to contain specific amounts of tissue cysts or bradyzoites and each formulation was back titrated in mice at the time of cat vaccination.

Five cats were vaccinated with either 10, 100 or 1000 bradyzoites which were in the form of NON PEPSIN TREATED tissue cysts. An additional 5 cats (per formulation) were vaccinated with either 10, 100 or 1000 bradyzoites which were in the form of PEPSIN DIGESTED cysts. The bradyzoite concentrations per dose were confirmed by back titration in mice. All cats were boostered with formulations containing 10, 100 or 1000 bradyzoites respectively at 21 days after primary vaccination. On day 43 post primary vaccination all cats were challenged with 10,000 bradyzoites of an oocyst-shedding T. gondii strain designated T-265. Five cats which were not vaccinated were challenged to serve as controls. Fecal samples were examined for oocyst shedding on days three to fourteen post vaccination and booster and for 14 days post challenge. Such fecal examination involved a microscopic evaluation of fecal samples for oocysts. In addition, the fecal samples were centrifuged and supernatant was removed and injected 1P into mice. Four weeks after injection, the mice were bled and checked for seroconversion to T. gondii. Seroconversion of mice injected with fecal supernatants (indirect agglutination titer is ≥1:40) indicates that there was at least one oocyst shed in the sample tested.

Table I shows that no oocysts were shed post vaccination or booster with any of the formulations. It is noted that five of the six vaccines produced protection in cats. One hundred percent (5/5) of control cats were infected and shed oocysts post challenge. Analysis of the results of the individual vaccines showed that the PEPSIN TREATED cyst vaccine produced more consistent protection. The dose containing 10 bradyzoites protected 40% of the cats post challenge. The vaccine containing 100 bradyzoites protected 80% of the cats. The vaccine containing 1000 bradyzoites protected 60% of the cats post challenge. Vaccines prepared from cysts which were NON PEPSIN TREATED, (bradyzoites were not released from the cysts and there was contamination with tachyzoites) protected as follows: 80% of the cats at a dose level of 10 bradyzoites, 0% at a dose level of 100 bradyzoites and 75% at a dose level of 1000 bradyzoites. This inconsistency is not desirable for a commercial product. Therefore, PEPSIN DIGESTION of tissue culture grown tissue cysts which results in release of the bradyzoites and destruction of the tachyzoites is preferable for commercial production of the vaccine.

The vaccine can be administered to cats in any desirable manner. Presently, an oral mode of administration is preferred. Dosages of 0.1mL to 2.0 mL and preferably 0.2 mL to 1.0 mL per cat can be employed. While the vaccines described hereinabove are liquid, it is within the purview of the skilled artisan to make and use lyophilized or frozen forms of the vaccine.

It has been demonstrated that tissue culture-grown T. gondii vaccine can immunize cats and thus protect them from shedding when they are later exposed to a virulent shedding strain. Additionally, bradyzoites prepared in tissue culture can be used for development of diagnostic assays. Table 1, hereinbelow further illustrates the invention.

## TABLE 1
## TISSUE CULTURE/VACCINATION-CHALLENGE STUDY

| Parameters of | Group 1 | Group 2 | Group 3 | Group 4 | Group 5 | Group 6 | Group 7 |
|---|---|---|---|---|---|---|---|
| Number of Cats | 5 | 5 | 5 | 5 | 5 | 5 | 5 |
| Day - 7 Titer | <40 | <40 | <40 | <40 | <40 | <40 | <40 |
| Day - 7 Oocyst Shedding (Prevacination) | 0/5 | 0/5 | 0/5 | 0/5 | 0/5 | 0/5 | 0/5 |
| Vaccine Type | No Treat | No Treat | No Treat | Pepsin Treat | Pepsin Treat | Pepsin Treat | N/A |
| Number of Bradyzoites | 10 | 100 | 1000 | 10 | 100 | 1000 | N/A |
| Oocyst Shedding Pose 1st Vac | 0/5 | 0/5 | 0/5 | 0/5 | 0/5 | 0/5 | N/A |
| Day 21 Titer | 8000<br><40<br>8000<br>8000<br>8000 | NS<br>NS<br><40<br>8000<br>4000 | 16000<br>8000<br>40<br>8000<br>8000 | 8000<br><40<br>40<br>40<br><40 | 8000<br>40<br>40<br>4000<br>4000 | 4000<br>8000<br>4000<br>4000<br>4000 | <40<br><40<br><40<br><40<br><40 |

NS = No Sample
N/A = Not Applicable as these are nonvaccinated controls
No Treat = No Treatment with Pepsin
Pepsin Treat = Treated with Pepsin

EP 0 687 471 A1

## TABLE 1 (CONTINUED)
## TISSUE CULTURE/VACCINATION-CHALLENGE STUDY

| Parameters of Study | Group 1 | Group 2 | Group 3 | Group 4 | Group 5 | Group 6 | Group 7 |
|---|---|---|---|---|---|---|---|
| Vaccine Type | No Treat | No Treat | No Treat | Pepsin Treat | Pepsin Treat | Pepsin Treat | N/A |
| Number of Bradyzoites | 10 | 100 | 1000 | 10 | 100 | 1000 | N/A |
| Oocyst Shedding Post Booster Dose | 0/5 | 0/3 | 0/4 | 0/5 | 0/5 | 0/5 | N/A |
| Day 43 Titer | 8000<br><40<br>8000<br>8000<br>8000 | NS<br>NS<br>4000<br>8000<br>8000 | 16000<br>8000<br>NS<br>3200<br>8000 | 8000<br><40<br>40<br>40<br>4000 | 800<br>40<br>4000<br>8000<br>8000 | 4000<br>8000<br>4000<br>8000<br>8000 | <40<br><40<br><40<br><40<br><40 |

NS = No Sample
No Treat = Not Treated with Pepsin
Pepsin Treat = Treated with Pepsin
N/A = Not Applicable as these are nonvaccinated controls

## TABLE 1 (CONTINUED)
## TISSUE CULTURE/VACCINATION-CHALLENGE STUDY

| Parameters of Study | Group 1 | Group 2 | Group 3 | Group 4 | Group 5 | Group 6 | Group 7 |
|---|---|---|---|---|---|---|---|
| Vaccine Type | No Treat | No Treat | No Treat | Pepsin Treat | Pepsin Treat | Pepsin Treat | N/A |
| Challenge Results Protection | 80% | 0% | 75% | 40% | 80% | 60% | 0% |
| Day 64 Titer | 8000 | NS | 16000 | 16000 | 8000 | 8000 | 8000 |
| | 8000 | NS | 8000 | 8000 | 8000 | 8000 | 16000 |
| | 8000 | 4000 | NS | 16000 | 8000 | 8000 | 8000 |
| | 8000 | 8000 | 800 | 8000 | 16000 | 8000 | 16000 |
| | 8000 | 8000 | 8000 | 8000 | 8000 | 8000 | 8000 |

NS = No Sample
N/A = Not Applicable as these are nonvaccinated controls
No Treat = Not Treated with Pepsin
Pepsin Treat = Treated with Pepsin
Age of Cats = 3 to 6 months
Challenge = approximately 10,000 bradyzoites grown in mice

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

**Claims**

1. A tissue culture produced T. gondii containing an immunogenically effective amount of bradyzoites for protection of cats against T. gondii.

2. The tissue culture of Claim 1 wherein the T. gondii is characterized by an oocyst negative phenotype.

3. The tissue culture of Claim 1 wherein the T. gondii is designated as T-263.

4. A process for preparing a tissue culture of T. gondii comprising:
   a) growing tachyzoites of the T. gondii in the tissue culture,
   b) keeping the tissue culture from being overgrown by tachyzoites and **developing** bradyzoites in tissue cysts, and
   c) killing residual tachyzoites.

5. The process of Claim 4 wherein the T. gondii is designated as T-263.

6. The process of Claim 4 wherein the tissue culture is selected from the group consisting of embryonic bovine lung cells, mouse astrocyte cells, human foreskin fibroblast cells and MRC-5 cells.

7. The process of Claim 4 wherein keeping the tissue culture from being overgrown by tachyzoites and **developing** bradyzoites in tissue cysts comprises adding positive antisera to the tissue culture.

8. The process of Claim 4 wherein killing residual tachyzoites comprises adding a proteolytic enzyme to the tissue culture before or after harvesting the tissue culture.

9. The process of Claim 8 wherein the proteolytic enzyme selected from the group consisting of pepsin, trypsin and chymotrypsin.

10. A vaccine containing the tissue culture produced T. gondii of Claim 1.

11. The vaccine of Claim 10 characterized in that it is live or modified live.

12. The vaccine of Claim 10 wherein the tissue culture produced T. gondii is derived from a mutant designated as T-263.

13. The vaccine of Claim 10 which is in a liquid, frozen or lyophilized state

14. A diagnostic kit for detecting T. gondii infection comprising the tissue culture of Claim 1.

15. A method of protecting cats from oocyst shedding by administering to said cats the vaccine of Claim 1.

# PARTIAL EUROPEAN SEARCH REPORT

Application Number

which under Rule 45 of the European Patent Convention shall be considered, for the purposes of subsequent proceedings, as the European search report

EP 95 10 8593

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | AMERICAN JOURNAL OF VETERINARY RESEARCH, vol. 52, no. 5, 1991 CHICAGO, pages 759-763, FRENKEL J.K. ET AL 'Prospective vaccine prepared from a new mutant of Toxoplasma gondii for use in cats' * page 761 - page 762 * --- | 1-15 | A61K39/002 C12N1/10 G01N33/569 |
| X,D | THE JOURNAL OF PARASITOLOGY, vol. 63, no. 6, 1977 LAWRENCE KANS., pages 1121-1124, HOFF R.L. ET AL 'Toxoplasma gondii cysts in cell culture: new biological evidence' * the whole document * --- | 4-9 | |
| X | WO-A-91 00740 (THE UNIVERSITY OF KANSAS) 1991 * page 12, line 1 - line 15 * * claims 1-4 * --- | 1-15 | |
|  | -/-- | | TECHNICAL FIELDS SEARCHED (Int.Cl.6) |
|  | | | A61K C12N |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 14 September 1995 | Fernandez y Branas,F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C07)

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X | THE JOURNAL OF PARASITOLOGY, vol. 77, no. 1, 1991 LAWRENCE, KANS., pages 126-132, LINDSAY, D.S. ET AL 'Examination of tissue cyst formation by Toxoplasma gondii in cell cultures using bradyzoites, tachyzoites, and sporozoites' * page 131 * --- | 4-9 | |
| A | EP-A-0 100 710 (KANSAS UNIVERSITY ENDOWMENT) 1984 * the whole document * --- | 1-15 | |
| X | JOURNAL OF IMMUNOLOGY, vol. 134, no. 5, 1985 BALTIMORE US, pages 3426-3431, KASPER, L.H. ET AL 'An unexpected response to vaccination with a purified mayor membrane tachyzoite antigen (P30) of Toxoplasma gondii' * page 3429 * * table V * --- | 14 | TECHNICAL FIELDS SEARCHED (Int.Cl.6) |
| A | INFECTION AND IMMUNITY, vol. 51, no. 1, 1986 WASHINGTON US, pages 147-156, JONES T.J. ET AL 'In vitro cultivation of Toxoplasma gondii Cysts in Astrocytes in the presence of gamma interferon' * the whole document * ----- | 1-15 | |

EP 95 10 8593

-C-

Remark : Although claim 15 is directed to a method of
treatment of the human/animal body (Article
52(4) EPC) the search has been carried out and
based on the alleged effects of the composition.